(19) Europäisches Patentamt
European Patent Office
Office européen des brevets

(11) Publication number: 0 259 159 A2

# (12) EUROPEAN PATENT APPLICATION

(21) Application number: 87307780.4

(22) Date of filing: 03.09.87

(51) Int. Cl.[4]: C 07 C 91/34
C 07 C 47/575, C 07 C 119/10

(30) Priority: 05.09.86 US 903813

(43) Date of publication of application:
09.03.88 Bulletin 88/10

(84) Designated Contracting States:
AT BE CH DE ES FR GB GR IT LI LU NL SE

(71) Applicant: SCHERING CORPORATION
2000 Galloping Hill Road
Kenilworth, New Jersey 07033 (US)

(72) Inventor: Babad, Esther
30 Conforti Avenue
West Orange New Jersey 07052 (US)

Carruthers, Nicholas
200 Springfield Avenue Apt. No. 1025
Springfield New Jersey 07081 (US)

Steinman, Martin
3, Tuxedo Drive
Livingston New Jersey 07039 (US)

(74) Representative: Ritter, Stephen David et al
Mathys & Squire 10 Fleet Street
London EC4Y 1AY (GB)

## (54) Method for the preparation of alpha1- [[(1,1-Dimethylethyl) amino] methyl]-4-hydroxy-1,3-benzenedimethanol, and intermediates used in its preparation.

(57) There is disclosed an improved process for preparing albuterol which comprises reacting a 5-(haloacetyl)-2-hydroxybenzaldehyde with l,l-dimethylethanamine in an organic solvent and in an inert atmosphere to form 5-[[(l,l-dimethylethyl)amino]acetyl]-2-hydroxybenzaldehyde and reducing the carbonyl functions of this last-named compound to the corresponding hydroxy groups to form albuterol.

EP 0 259 159 A2

## Description

### METHOD FOR THE PREPARATION OF ALPHA[1]-[[(l,l-DIMETHYLETHYL)AMINO]METHYL]-4-HYDROXY-l,3-BENZENEDIMETHANOL, AND INTERMEDIATES USED IN ITS PREPARATION

$\alpha^1$-[[(l,l-Dimethylethyl)amino]methyl]-4-hydroxy-l,3-benzenedimethanol, a substance known as albuterol and salbutamol, is a potent long-lasting β-adrenoceptor stimulant that is orally effective and shows highly selective action on bronchial smooth muscle. Albuterol is indicated for the relief of bronchospasm in patients with reversible airway disease.

Albuterol has been prepared (Irish Patent Specification No. 3l39l, August 9, l972) from the appropriate acetophenone derivative methyl 5-(bromoacetyl)-2-hydroxybenzoate, by condensation with N-(l,l-dimethylethyl)benzenemethanamine in the presence of base to form the ketonic ester, methyl 5-[[(l,l-dimethylethyl)(phenylmethyl)-amino]acetyl]-2-hydroxybenzoate. The ketonic ester is reduced with lithium aluminum hydride in tetrahydrofuran under nitrogen to yield $\alpha^1$-[[(l,l-dimethylethyl)(phenylmethyl)amino]methyl]-4-hydroxy-l,3-benzenedimethanol which is subsequently debenzylated with hydrogen in the presence of palladium-on-carbon catalyst to produce albuterol.

We have found that albuterol can be conveniently prepared in good yield by reacting 5-(haloacetyl)-2-hydroxybenzaldehyde with l,l-dimethylethanamine followed by reduction of the intermediate ketone compound, 5-[[(l,l-dimethylethyl)amino]acetyl]-2-hydroxybenzaldehyde. The synthesis does not require protection of any of the reactive functional groups on either of the reactants, for example the hydroxy or aldehyde function of the 2-hydroxybenzaldehyde starting material or the amino function of the l,l-dimethylethanamine.

The present invention provides a method for the preparation of albuterol in good yield without the need to protect any of the functional groups on any of the reagents.

The process of this invention is a novel multistep process in which certain individual steps are novel and in which certain of the intermediates are novel.

In a preferred embodiment described below, albuterol is produced by following the reaction sequence shown below:

II

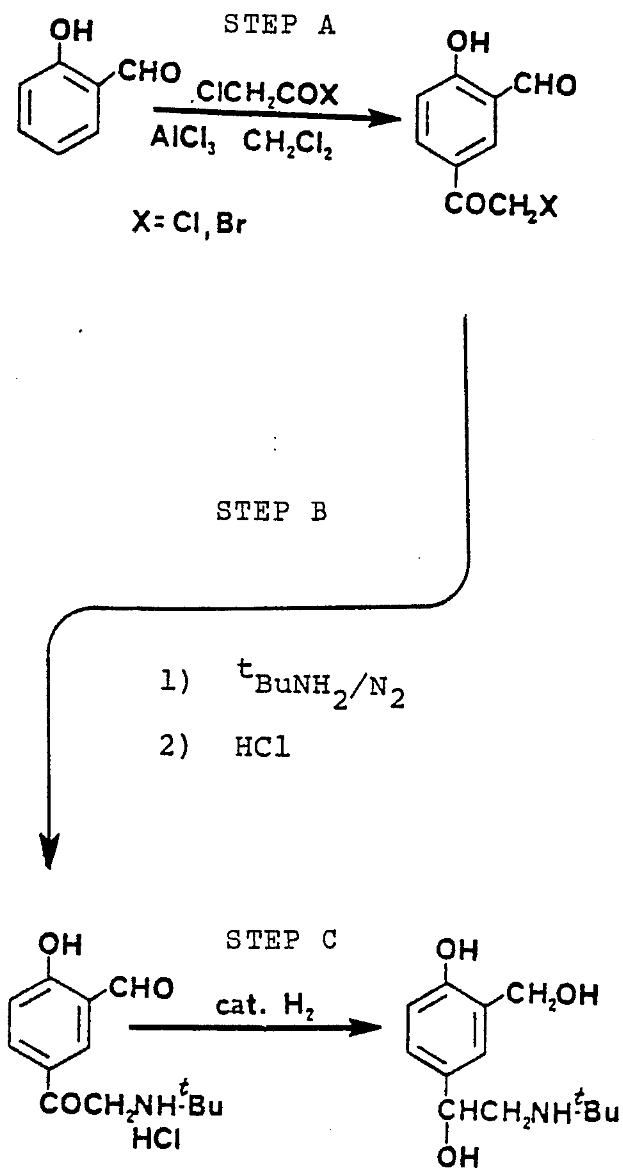


III

IV

I

The invention itself is of course broader than, and has more aspects than, this simple reaction scheme. The main process features however comprise the conversions III→IV→I and IV→I.

The invention therefore provides a method for the preparation of $\alpha^1$-[[(l,l-dimethylethyl)amino]methyl]-4-hydroxy-l,3-benzenedimethanol (I) which comprises:

l) reacting a 5-(haloacetyl)-2-hydroxybenzaldehyde compound of the formula

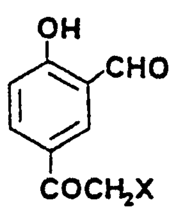

III

wherein X is bromo or chloro with an excess of l,l-dimethylethanamine, or with one equivalent of an organic amine R-$NH_2$ having a $pK_b$ value lower than that of ammonia (4.75) and then with an excess of l,l-dimethylethanamine, in an organic solvent and under an inert atmosphere to form 5-[[(l,l-dimethylethyl)amino]acetyl]-2-hydroxy-benzaldehyde, a compound of the formula

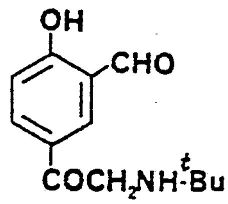

IV

and 2) reducing the carbonyl functions in the compound of formula IV to hydroxy groups to produce compound I.

The organic amine R-$NH_2$ is conveniently an alkylamine having I-I2 carbon atoms (preferably I-6, e.g. 2-4), an aralkylamine having 7-I2 carbon atoms (preferably 7), or a cycloalkylamine having 5-7 carbon atoms (preferably 6). I,I-Dimethylethanamine itself falls within this group, since it is an alkylamine having 4 carbon atoms.

Reaction of the compound of the formula III with the amine R-$NH_2$ yields a Schiff base of formula

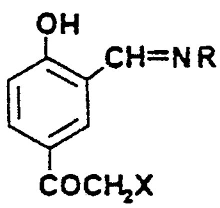

, e.g.

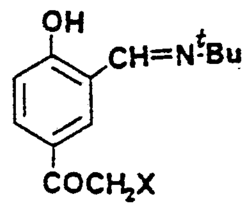

which if desired can be isolated (although it is not necessary to do so). Further reaction with I,I-dimethylethanamine yields a Schiff base of formula

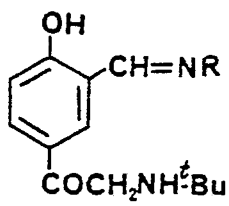

, e.g.

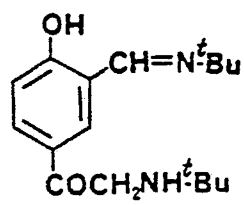

which if desired can also be isolated (although again it is not necessary to do so). Hydrolysis of this last compound with aqueous acid yields the compound of formula IV, usually precipitated as the acid addition salt.

The individual steps for the preparation of the compound of formula IV will now be described in more detail, especially in relation to preferred embodiments.

Step A

5-(Haloacetyl)-2-hydroxybenzaldehyde (III) is prepared by reacting 2-hydroxybenzaldehyde (II) with a haloacetylhalide under Friedel-Crafts conditions. Such Friedel-Crafts procedures are known to those skilled in the art and are described in the chemical literature, for example, "Friedel-Crafts and Related Reactions", George A. Olah (Ed.), Interscience Publishers (4 Volumes). Haloacetylhalide compounds that can be utilized as the starting material include bromoacetylchloride, chloroacetylchloride, and the like. If the halogen X in the haloacetyl moiety is different from that in the aluminum halide, at least partial exchange may occur; however, this is of no consequence. In carrying out this process any Lewis acid can be employed as the catalyst; aluminum trichloride is the preferred catalyst. Usually the reaction will be carried out in an organic solvent inert towards the reactants, for example, dichloromethane, I,2-dichloroethane, nitrobenzene, or chlorobenzene. The preferred solvents include dichloromethane and nitrobenzene. The reaction is carried out at elevated temperature, preferably at a temperature of from 40° to I00°C., e.g. in dichloromethane under reflux. Isolation of the resulting 5-(haloacetyl)-2-hydroxybenzaldehyde (III) can be carried out by employing conventional procedures well known in the art, such as column chromatography, recrystallization, and the like. Any ortho isomer formed during the reaction is removed by recrystallization.

5-(Haloacetyl)-2-hydroxybenzaldehyde may also be prepared in a two-step procedure, by means of acetylation and then halogenation.

Step B

5-[[(I,I-Dimethylethyl)-amino]acetyl]-2-hydroxybenzaldehyde (IV) is prepared by reacting 5-(haloacetyl)-2-hydroxybenzaldehyde (III) with I,I-dimethylethanamine. In carrying out this reaction, the I,I-dimethylethanamine is present in excess, for example up to I0 to I and preferably about 3 to I of I,I-dimethylethanamine to (III).

The reaction of I,I-dimethylethanamine with the halo derivative (III) initially produces the Schiff base having the structural formula V which may if desired be isolated, although it is not necessary to do so:

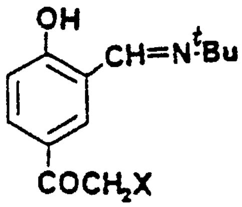

V.

Further reaction of V with I,I-dimethylethanamine produces the compound of structural formula VI:

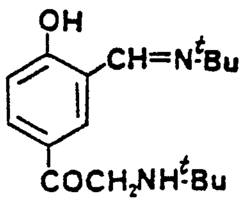

VI.

Treatment of the Schiff base VI with aqueous acid leads to hydrolysis of said compound and precipitation of IV as the acid addition salt.

In order to obtain the desired compound IV in good yield it is essential that the reaction be carried out under an inert atmosphere, for example under argon or nitrogen or the like. The reaction is preferably carried out by suspending the haloacetyl compound III in an organic solvent and treating the suspension with an excess of I,I-dimethylethanamine at a temperature from room temperature to reflux of the mixture. Examples of organic solvents that can be utilized in this reaction include 2-propanol, I,I-dimethylethanol, 2-methyl-I-propanol, 2-butanol, and the like. The preferred solvent is 2-propanol.

The amino compound IV is conveniently isolated as the acid addition salt. Acids that can be employed to form these salts are any suitable inorganic or organic acid, for example, hydrochloric, sulfuric, and 4-methylbenzenesulfonic acids.

We have discovered that, if the reaction of the haloacetyl compound (III) with I,I-dimethylethanamine is not carried out under an inert atmosphere, the compound of structural formula VI undergoes the following reaction owing to the presence of oxygen:

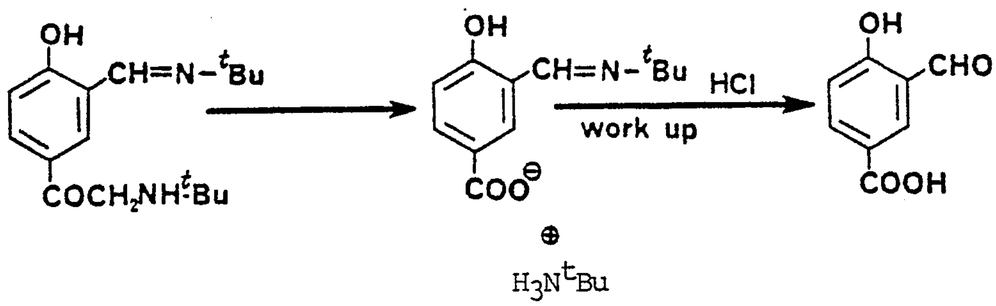


VI VII VIII

This undesirable side reaction can be prevented by running the reaction under an inert atmosphere.

Step C

The keto and aldehyde groups of the acid addition salt from Step B are then reduced to alcoholic groups by conventional methods well known to persons skilled in the art. In the preferred method the keto and aldehyde groups are reduced by catalytic hydrogenation. Suitable catalysts include palladium on carbon, or other

platinum metals, and the like. Such methods are known to those skilled in the art and are described in "Catalytic Hydrogenation Over Platinum Metals", Paul N. Rylander, Academic Press, 1967, and "Catalytic Hydrogenation In Organic Synthesis", Paul N. Rylander, Academic Press 1979. The hydrogenation can be carried out by dissolving the acid addition salt, compound (IV), in an appropriate solvent, such as anhydrous methanol, ethanol, 2-propanol, and the like. After addition of base, such as sodium methoxide, the hydrogenation is carried out in a suitable apparatus, for example a Parr apparatus. The resulting product is isolated by conventional techniques.

Alternatively, the keto and aldehyde groups can be reduced to alcohol groups with other reagents such as suitable hydrides, for example sodium borohydride, lithium borohydride or amino boranes, for example, 1,1-dimethylethylaminoborane, in an organic solvent. Methods and reagents are described in "Reductions in Organic Chemistry", Milos Hudlicky, Ellis Horwood, 1984.

$\alpha^1$-[[(1,1-Dimethylethyl)amino]methyl]-4-hydroxy-1,3-benzenedimethanol is conveniently isolated as the free base or as the acid addition salt. Acids that can be employed to form the acid addition salt are suitable inorganic and organic acids such as hydrochloric, sulfuric, 4-methylbenzenesulfonic acid, and the like.

EXAMPLE I

1. Step A

5-(BROMOACETYL)-2-HYDROXYBENZALDEHYDE

Preparation (a)

Aluminum trichloride (333g) is suspended in dichloromethane (350 ml) with stirring and heated to reflux temperature. To this slurry is added bromoacetyl chloride (99g) in dichloromethane (75ml) and after 30 minutes 2-hydroxybenzaldehyde (61.1g) in dichloromethane (75ml) is added dropwise. The reaction mixture is refluxed for 16 hours and then cautiously poured onto ice (3kg), deionized water (300ml) and dichloromethane (500ml) with stirring. Once the addition is complete the mixture is stirred for a further 15 minutes and the pH of the aqueous phase adjusted to pH 1-2 (pH paper) with concentrated hydrochloric acid if necessary. The organic layer is separated and the aqueous layer extracted with dichloromethane (3 × 250ml). The organic portions are combined, washed with deionized water (2 × 250ml) and saturated sodium chloride solution (500ml). The dichloromethane solution is dried over anhydrous magnesium sulfate and the dried solution evaporated under reduced pressure to give an oily solid (107g). The solid is slurried with a mixture of dichloromethane (20ml) and diethylether (180ml) and filtered off. The solid obtained is dried *in vacuo* at 50° to give 83g of a mixture (6:1) of the title compound and 5-(chloroacetyl)-2-hydroxybenzaldehyde.

NMR ($CDCl_3$): δ 4.35 (s, $CH_2$-Br)
4.65 2H (s, $CH_2$-Cl)
4.65 2H (s, $CH_2$-CL
7.05 (1H, d, J=10Hz)
8.00-8.30 (2H, m)
9.90 (1H, s)
11.50 (1H, s)

Preparation (b)

Aluminum tribromide (26.7g) in dibromomethane (100ml) is treated with bromoacetylbromide (5.1g) with stirring. After 30 minutes this solution is treated with 2-hydroxybenzaldehyde (2.4g). The reaction mixture is then heated at 80-90° (external oil bath) for 18 hours and cautiously poured onto ice (800g) with stirring. The organic layer is separated and the aqueous layer extracted with dichloromethane (2 × 100ml). The organic portions are combined, washed with saturated sodium chloride solution (2 × 250ml) and dried over anhydrous magnesium sulfate. The dried solution is evaporated under reduced pressure to afford a brown oil. Purification is achieved by chromatography on silica gel eluting with hexane:ethylacetate mixtures to give 0.46g of the title compound as a light crystalline solid.

NMR ($CDCl_3$): δ 4.4 (2H,s)
7.05 (1H, d, J=10Hz)
8.00-8.03 (2H, m)
9.90 (1H, s)
11.40 (1H, s)

IR (KBr): ν 1650 $cm^{-1}$
UV ($CH_3CN$): $\lambda_{max}$ 243nm (ε=24,082)
Found: C, 44.47; H, 2.83; Br, 32.24%.

2. 5-(CHLOROACETYL)-2-HYDROXYBENZALDEHYDE

Preparation (a)

Aluminum trichloride (l00g) is suspended in dichloromethane (l25ml) with stirring and the mixture is heated to reflux temperature. This slurry is treated with chloroacetylchloride (28.2g) and after 30 minutes with 2-hydroxybenzaldehyde (24.4g) dropwise. Once addition is complete the reaction mixture is heated at reflux for a further l8 hours and poured cautiously onto ice (lkg), deionized water (l00ml) and dichloromethane (l00ml). The solution is stirred for l5 minutes and the pH of the aqueous layer adjusted to pH l-2 (pH paper) by addition of concentrated hydrochloric acid if necessary. The organic layer is separated and the aqueous layer extracted with dichloromethane (3 × l50ml). The organic portions are combined, washed with deionized water (2 × 250ml), saturated sodium chloride solution (500ml) and dried over anhydrous magnesium sulfate. The dried solution is evaporated under reduced pressure to give an oily solid. The solid is dissolved in hot 2-butanone (70ml) from which the title compound crystallizes upon cooling. The title compound (l6g) is isolated by suction filtration.

NMR ($CDCl_3$): δ 4.65 (2H,s)
7.06 (lH, d, J=l0Hz)
8.00-8.26 (2H, m)
9.95 (lH, s)
ll.45 (lH, s)

IR (KBr): ν l650 $cm^{-1}$
UV ($CH_3CN$): $\lambda_{max}$ 24lnm (ε=27,952)
Found: C, 54.4l; H, 3.45; Cl, l7.72%.

Preparation (b)

Aluminum trichloride (l80g) is suspended in dichloromethane (500ml) and heated with stirring to reflux temperature. To this slurry is added phosphoryl chloride (l2.5ml) in dichloromethane (l2.5ml), chloroacetylchloride (40.5ml) in dichloromethane (40ml) and after 60 minutes 2-hydroxybenzaldehyde (l8.8ml) in dichloromethane (20ml). The reaction mixture is refluxed for 24 hours and then cautiously added to ice (l.5kg), deionized water (500ml) and dichloromethane (500ml) with stirring. Once addition is complete the mixture is agitated for 30 minutes and the pH of the aqueous phase adjusted to pH l-2 (pH paper) by addition of concentrated hydrochloric acid, if necessary. The organic layer is separated and the aqueous layer extracted with dichloromethane (2 × 200ml). The organic portions are combined, washed with deionized water (2 × 200ml) and concentrated in vacuo (to approximately l50ml). The concentrated solution is diluted with methylbenzene (300ml) and evaporated in vacuo (to approximately l00ml) whereupon l0.4g of the title compound is isolated by suction filtration. Further purification is achieved by recrystallization from 2-butanone.

Step B

l. 5-[[(l,l-DIMETHYLETHYL)AMINO]ACETYL]-2-HYDROXYBENZALDEHYDE HYDROCHLORIDE

Preparation (a)

5-(Bromoacetyl)-2-hydroxybenzaldehyde (l0.8g) is suspended in 2-propanol (40ml) with stirring under an inert atmosphere and treated with l,l-dimethylethanamine (l4ml). This solution is heated at reflux temperature for 2 hours and treated with a mixture of l2M hydrochloric acid (ll.lml) and 2-propanol (9ml). After a further l8 hours' stirring and cooling to room temperature a solid is isolated by filtration and dried in vacuo at 50°. The dried material is slurried with 2-propanol (l00ml), stirred for l8 hours, filtered off and dried in vacuo at 50°. In this manner 7g of the title compound is obtained as a free-flowing powder.

NMR ($(CD_3)_2SO$): δ l.4 (9H,s)
4.65 (2H, s)
7.3 (lH, d, J=l0 Hz)
8.l-8.4 (2H, m)
l0.3 (lH, s)

IR (KBr): ν l670$cm^{-1}$
UV ($CH_3CN$): $\lambda_{max}$ 243nm (ε=22,4ll).
Found: C, 57.43; H, 6.63; N, 4.88.

Preparation (b)

5-(Chloroacetyl)-2-hydroxybenzaldehyde (l0g) is suspended in 2-propanol (60ml) with stirring at room temperature under an inert atmosphere. This slurry is treated with l,l-dimethylethanamine (l6.4ml) and heated at reflux for l hour and then treated with a mixture of l2M hydrochloric acid (l3.0ml) and 2-propanol (20ml). The reaction mixture is allowed to cool to room temperature and stirred for a further l8 hours. Filtration of the reaction mixture affords the title compound (6.2g after washing with diethylether (50ml) and drying in vacuo at 50°).

Preparation (c)

5-(Bromoacetyl)-2-hydroxybenzaldehyde (l09g) is suspended in 2-propanol (920ml) and cooled to -3° C with stirring. The reaction mixture is treated with l,l-dimethylethanamine (29.24g) and stirred at -3° to 0° C for 30 minutes whereupon precipitation occurs. The reaction mixture is allowed to warm to room temperature, stirred for 90 minutes, and then cooled to l0° C and filtered. The solid obtained is washed with ice cold 2-propanol (2 × l00ml) and dried in vacuo at 55°C for l8 hours providing 89g of 2-bromo-l-[3-[[(l,l-dimethylethyl)imino-]methyl]-4-hydroxyphenyl]ethanone.

NMR ($CDCl_3$:$(CD_3)_2SO$): δ l.43 (9H,s)
4.45 (2H, s)
6.80 (lH, d, J=l0 Hz)
7.75-8.l0 (2H, m)
8.45 (lH, brs)

IR (KBr): ν l625 $cm^{-1}$
UV ($CH_3CN$): $\lambda_{max}$ 250 nm (ε=l8,854).
Found: C, 52.68; H, 5.35; N, 4.77; Br, 26.77%.

2-Bromo-l-[3-[[(l,l-dimethylethyl)imino] methyl]-4-hydroxyphenyl]ethanone (ll.93g) is suspended in 2-propanol (l00ml) under an inert atmosphere and treated with l,l-dimethylethanamine (8.8g) and a further portion of 2-propanol (50ml). The solution is heated at 80° until all the starting material is consumed. The reaction mixture is cooled to -l0°, and treated with l2M hydrochloric acid (llml) and 2-propanol (50ml). Stirring is continued for an additional l8 hours during which time the solution warms to room temperature. A solid is isolated from the solution, slurried in 2-propanol (50ml), filtered and dried in vacuo at 50° giving 5.0g of 5-[[(l,l-dimethylethyl)amino]acetyl]-2-hydroxybenzaldehyde hydrochloride.

Preparation (d)

2-(Chloroacetyl)-2-hydroxybenzaldehyde (lg) is suspended in 2-propanol (l0ml) with stirring at room temperature. The slurry is treated with l,l-dimethylethanamine (0.63ml) and stirred for l8 hours. Filtration of the reaction mixture affords 0.4g 2-chloro-l-[3-[[l,l-dimethylethyl)imino]methyl-4-hydroxyphenyl]ethanone as a yellow solid.

NMR ($CDCl_3$): δ l.4 (9H,s)
4.55 (2H, s)
6.85 (lH, d, J=l0 Hz)
7.70-7.90 (2H, m)
8.23 (lH, brs)

IR (KBr): ν l600 $cm^{-1}$
UV ($CH_3CN$): $\lambda_{max}$ 249 nm (ε=23,563).
Found: C, 6l.50; H, 6.48; N, 5.26; and Cl, l3.90%.

2-Chloro-l-[3-[[(l,l-dimethylethyl)imino] methyl]-4-hydroxyphenyl]ethanone (l0g) is suspended in 2-propanol (60ml) with stirring at room temperature under an inert atmosphere. This slurry is treated with l,l-dimethylethanamine (l6.4ml) and heated at reflux for l hour and then treated with a mixture of l2M hydrochloric acid (l3.0ml) and 2-propanol (20ml). The reaction mixture is allowed to cool to room temperature and stirred for an additional l8 hours. Filtration of the reaction mixture affords (6.2g) of 5-[[(l,l-dimethylethyl)amino]acetyl]-2-hydroxybenzaldehyde hydrochloride after washing with diethylether (50ml) and drying in vacuo at 50°.

2. 5-[[(l,l-DIMETHYLETHYL)AMINO]ACETYL]-2-HYDROXYBENZALDEHYDE 4-METHYLBENZENESULFONATE

Preparation (a)

5-(Bromoacetyl)-2-hydroxybenzaldehyde (l8.0g) is suspended in 2-propanol (l00ml) at 0°, under an inert atmosphere, and treated with l,l-dimethylethanamine (23.4ml), and the reaction mixture is allowed to warm to room temperature. Stirring is continued for a further 50 minutes and the solution is treated with a mixture of 4-methylbenzenesulfonic acid monohydrate (42.3g), 2-propanol (35ml) and deionized water (20ml). This solution is stirred for a further l8 hours, filtered, and the residue washed with diethylether (50ml) and then dried in vacuo at 50°. In this manner the title compound (20.0g) is obtained as a free-flowing powder.

NMR ($(CD_3)_2SO$): δ l.37 (9H,s)
2.30 (3H,s)
3.37 (2H,s)
7.l2, 7.50 (4H, $A_2B_2$, J=9Hz)
7.20 (lH, d, J=l0Hz)
8.25 (lH, dd, J=2, l0Hz)
8.40 (lH, d, J=2 Hz)

Preparation (b)

5-(Chloroacetyl)-2-hydroxybenzaldehyde (7.9g) is suspended in 2-propanol (50ml) under an inert atmosphere and treated, with stirring, with l,l-dimethylethanamine (l3 ml). The mixture is heated at reflux for 2 hours and treated with a mixture of 4-methylbenzenesulfonic acid monohydrate (23.5g), 2-propanol (l6ml) and deionized water (8ml). The solution is stirred for a further l8 hours and filtered, and the residue washed with diethylether (50ml) and dried in vacuo at 50°. The solid isolated is washed with 2-propanol (l00ml) and dried in vacuo at 50° giving the title compound (9.02g).

3.5-[[(l,l-DIMETHYLETHYL)AMINO]ACETYL]-2-HYDROXYBENZALDEHYDE HEMISULFATE

5-(Bromoacetyl)-2-hydroxybenzaldehyde (l0g) is suspended in 2-propanol (60ml) under an inert atmosphere and cooled to 0°. To the stirring slurry is added l,l-dimethylethanamine (l3 ml) and the reaction mixture is allowed to warm to room temperature and stirred for 50 minutes. The solution is then treated with a mixture of concentrated sulfuric acid (3.5ml), 2-propanol (6.5ml) and deionized water (5ml). The reaction mixture is stirred for a further l8 hours whereupon the title compound (l0.2g) is isolated by filtration and dried in vacuo at 50°.

NMR ($(CD_3)_2SO$): δ l.4l (9H,s)
4.68 (2H,s)
7.l5 (lH, d, J=l0Hz)
8.l-8.45 (2H, m)
l0.35 (lH, s)

l.Step C

α[1]-[[(l,l-DIMETHYLETHYL)AMINO]METHYL]-4-HYDROXY-l,3-BENZENEDIMETHANOL

Preparation (a)

5-[[(l,l-Dimethylethyl)amino]acetyl]-2-hydroxybenzaldehyde hydrochloride (0.774g) is dissolved in anhydrous methanol (20ml) at ice-bath temperature. To the solution is added sodium methoxide (0.l54g), with stirring, and after 5 minutes 5% palladium on carbon (0.23g). The mixture is hydrogenated at room temperature, at 5l5 kPa (60 psig) of hydrogen in a Parr apparatus. After l0 hours the reaction mixture is filtered through a pad of Celite® and the filtrate evaporated to give the title compound (0.6g).

In a similar manner the reaction mixture may be filtered before addition of catalyst and again after hydrogenation is complete.

NMR ($CD_3OD$): δ l.3 (9H, s)
2.9 (2H, m)
4.65-4.90 (3H, m)
6.80 (lH, d, J=9Hz)
7.20 (lH, dd, J=2, 9Hz)
7.45 (lH, d, J=2Hz)

Preparation (b)

5-[[(l,l-Dimethylethyl)amino]acetyl]-2-hydroxybenzaldehyde 4-methylbenzenesulfonate (6.l8g) is dissolved in anhydrous methanol (l00ml) and the solution cooled in an ice bath. To the cool, stirred solution is added sodium methoxide (0.93g) and then after l0 minutes 5% palladium on carbon (l.3g). The reaction mixture is hydrogenated at room temperature at 5l5 kPa (60 psig) of hydrogen in a Parr apparatus. After l6 hours the reaction mixture is filtered thorough a pad of Celite® and evaporated. Sodium 4-methylbenzene sulfonate is removed from the product by dissolving the crude material in methanol and triturating with ethylacetate as many times as necessary. In this way the title compound (3.lg) is isolated as a homogeneous solid.

2. α[1]-[[(l,l-DIMETHYLETHYL)AMINO]METHYL]-4-HYDROXY-l,3-BENZENEDIMETHANOL HEMISULFATE

α[1]-[[(l,l-Dimethylethyl)amino]methyl]-4-hydroxy-l,3-benzenedimethanol (5.8g) is dissolved in deionized water (8ml) containing concentrated sulfuric acid (0.68ml). The solution is stirred for 60 minutes, treated with ethanol (60ml), stirred for l8 hours and treated with a further portion of ethanol (60ml), and the title compound (3.35g) isolated by filtration as a white powder.

NMR ($(CD_3)_2SO$): δ l.25 (9H,s)
2.72-2.98 (2H, m)
4.49 (2H,s)
4.83 (lH, m)
6.75 (lH, d, J=l0Hz)
7.l0 (lH, dd, J=2, l0Hz)
7.32 (lH, d, J=2Hz)

## Claims

I. A method for the preparation of $\alpha^1$-[[(I,I-dimethylethyl)amino]methyl]-4-hydroxy-I,3-benzenedimethanol (I) which comprises:

I) reacting a 5-(haloacetyl)-2-hydroxybenzaldehyde compound of the formula

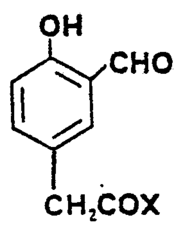

III

wherein X is bromo or chloro with an excess of I,I-dimethylethanamine, or with one equivalent of an organic amine $R\text{-}NH_2$ having a $pK_b$ value lower than that of ammonia (4.75) and then with an excess of I,I-dimethylethanamine, in an organic solvent and under an inert atmosphere to form 5-[[(I,I-dimethylethyl)amino]acetyl]-2-hydroxy-benzaldehyde, a compound of the formula

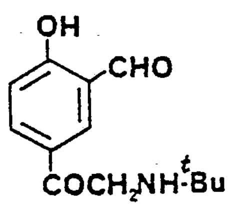

IV

and 2) reducing the carbonyl functions in the compound of formula IV to hydroxy groups to produce compound I.

2. A method as claimed in claim I wherein step I is carried out under an inert atmosphere of nitrogen or argon, and the organic solvent is selected from propan-2-ol, I,I-dimethylethanol, 2-methyl-I-propanol and 2-butanol.

3. A method as claimed in claim I or claim 2 wherein in Step I I,I-dimethylethylamine is reacted directly with compound III in a ratio of up to I0 to I, preferably about 3 to I.

4. A method as claimed in any of claims I to 3 wherein the compound of formula IV is isolated as an acid addition salt, e.g. as the hydrochloric acid salt, as the 4-methylbenzenesulfonic acid salt, or as the hemisulfate acid salt.

5. A method as claimed in any of claims I to 4 wherein the compound of formula IV is reduced by catalytic hydrogenation, e.g. with palladium on carbon.

6. A method as claimed in any of claims I to 5 wherein compound I is isolated as an acid addition salt, e.g. as the hemi-sulfate acid salt.

7. The compound 5-[[(I,I-dimethylethyl)amino]acetyl]-2-hydroxybenzaldehyde and acid addition salts thereof.

8. A compound of the formula

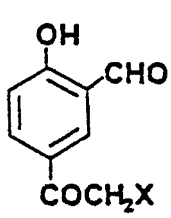

wherein X is bromo or chloro.

9. A compound of the formula

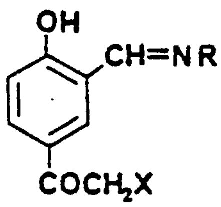

wherein X is bromo or chloro and R is an alkyl group having from I to I2 carbon atoms, an aralkyl group having from 7 to I2 carbon atoms, or a cycloalkyl group having 5 to 7 carbon atoms, in particular the compound of the formula

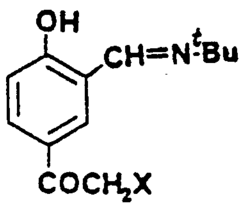

I0. A method for the preparation of $\alpha^1$[[I,I-dimethylethyl)amino]-methyl]-4-hydroxy-I,3-benzenedimethanol (I) which comprises reducing the carbonyl functions of 5-[[(I,I-dimethylethyl)amino]acetyl]-2-hydroxybenzaldehyde.

II. A method as claimed in claim I0 wherein the compound of formula IV is reduced by catalytic hydrogenation, preferably with palladium on carbon.

I2. A method for the preparation of a compound of the formula

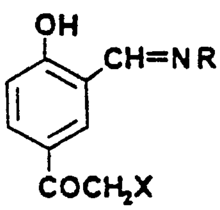

, especially

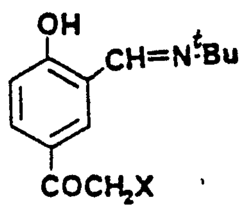

wherein X is bromo or chloro and R is as defined in claim 9, which comprises reacting a 5-(haloacetyl)-2-hydroxybenzaldehyde compound of the formula

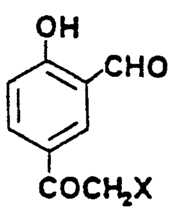

III

wherein X is bromo or chloro, with one equivalent of an amine of the formula $R-NH_2$, where R is as defined in claim 9, especially with one equivalent of I,I-dimethylethanamine, in an organic solvent in an inert atmosphere, preferably in an organic solvent selected from propan-2-ol, I,I-dimethylethanol, 2-methyl-I-propanol and 2-butanol.